# EUROPEAN PATENT APPLICATION

(11) **EP 3 266 428 A1**
(43) Date of publication of application: **10.01.2018**
(21) Application number: 17179231.0
(22) Date of filing: 03.07.2017
(51) Int. Cl.: A61F 13/00, A61F 13/06, A61F 13/08

(54) **ELASTIC BANDAGE, PARTICULARLY FOR USE IN THE TREATMENT OF PHLEBO-LYMPHOEDEMA**

(30) Priority: 05.07.2016 IT 202016069689 U
(71) Applicant: Cizeta Medicali S.p.A., 20012 Cuggiono (Milano) (IT)
(72) Inventor: VALLARINO GANCIA, Edoardo, 10123 TORINO (IT)
(74) Representative: Rambelli, Paolo

(57) **Abstract**

An elastic bandage for realizing an elastic compression or elastic containment bandaging formed by a strip of fabric with longitudinal extension (2) and comprising on at least one of its surfaces a plurality of punctiform elements (8) of elastomeric material separated and spaced from each other.

The elastic bandage is particularly useful in the treatment of phlebolymphoedema.

## Description

The present invention refers to an elastic bandage, of the type commonly used for bandaging a limb.

In particular, the invention relates to an improved elastic bandage, particularly useful in the active treatment of phlebolymphoedema or edema from venous lymphatic insufficiency, with the realization of an elastic compression bandage or elastic containment bandage.

The primary purpose of the invention is to provide an improved bandage that allows for better bandage tightness compared to a conventional multilayered short-stretch bandage.

Another object of the invention is to provide an elastic bandage which, at the same tightness, allows for greater freedom of movement for the patient compared to the conventional multilayered short-stretch bandage.

Another object of the invention is to provide a bandage that allows a bandaging to maintain its rigidity for a longer period of time compared to the conventional short-stretch bandage.

With a view to these and other objects which will become apparent from the following description, the object of the invention is an elastic bandage, characterized in that it presents, on at least one of its surfaces, a plurality of punctiform elements of elastomeric material separated and spaced from each other, as defined in the claims below.

Further advantages and features of the bandage according to the invention will become apparent from the detailed description that follows, made with reference to the appended drawings, provided by way of non-limiting example, wherein:
- figure 1 is a perspective view of a bandage according to the invention, partially wound in a roll, and
- figure 2 is a top view of a section of the elastic bandage, and

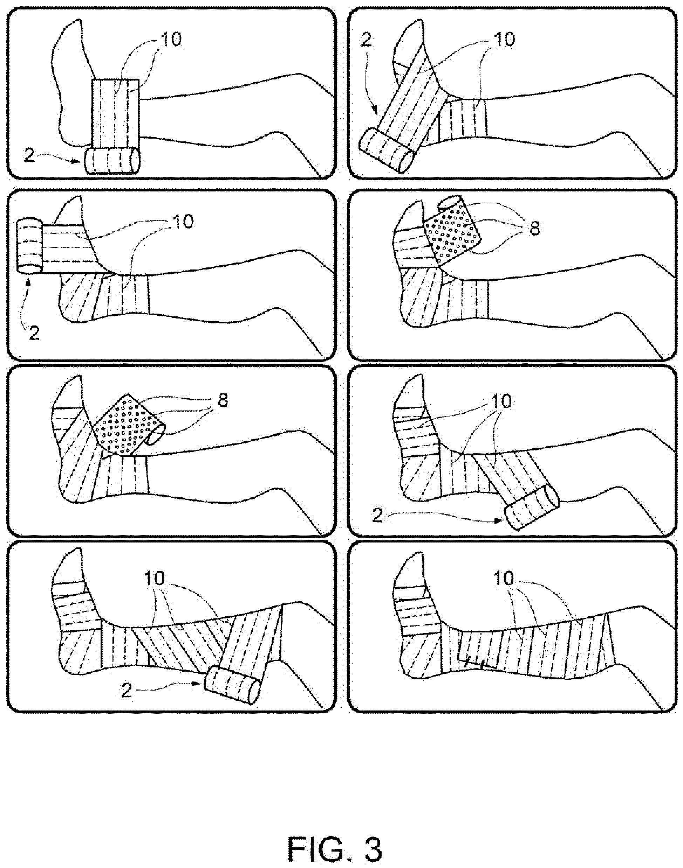

- figure 3 is a schematic representation of the bandaging steps carried out with the bandage according to the invention.

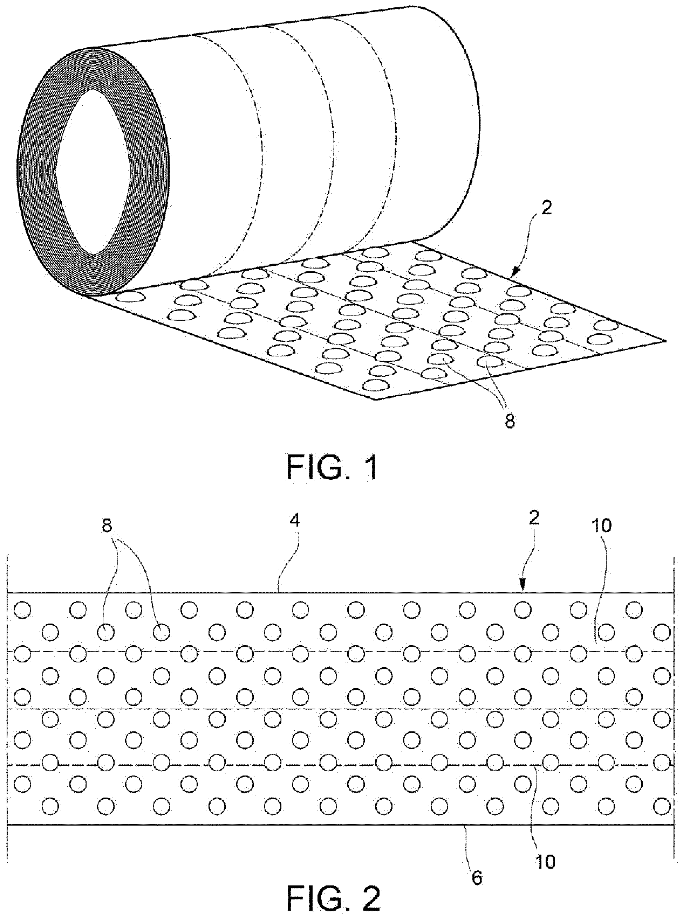

With reference to the drawings, figure 1, which is an example of a preferred embodiment, illustrates an elastic bandage indicated as a whole at 2, comprised, in a manner known per se, of an elongated strip wound in a roll with one end extended.

Preferably, this is a short-stretch bandage, i.e. having elastic elongation ranging from 30 to 70% and more preferably from 25 to 45% and even more preferably from 30 to 40%.

Preferably, the fabric is a double-weave fabric with cotton weft threads and polyamide warp threads with longitudinal selvage 4 and 6.

The choice of cotton and polyamide materials, although preferred, is however not to be understood as binding for the purposes of the invention. In addition, the relative ratio of cotton/polyamide may vary within broad limits in order to achieve the desired or preferred elongation characteristics.

The distinctive feature of the invention consists in the fact that the bandage has on at least one of its surfaces a plurality of punctiform elements, indicated at 8, of an elastomeric material, adhered to the surface of the bandage and spaced apart and separated from each other. The "punctiform elements", hereinafter also referred to as "dots", are small, lenticular formations with convex surfaces projecting from the fabric constituting the bandage, the contour of which may, however, vary widely, being not limited to the circular form illustrated in the drawings. Thus, included in the term "punctiform elements" or dots are polygonal or irregularly shaped formations.

The preferred material for making the dots is an elastomeric silicone (or polysiloxane), including, for example, polydimethylsiloxane, suitable for medical use. In particular, two-component tin-free silicones are used.

The size of the dots may vary widely depending on the size of the bandage and may thus be from about 1 mm to 6 mm, preferably from 2 to 5 mm, with a preferred size (or diameter) on the order of 3 or 5 mm.

The dots are preferably arranged according to an orderly grid, the configuration of which may vary, but preferably consisting of rows extending in the longitudinal direction of the bandage and being spaced from each other in the cross direction of the band, with constant pitch or distance. In each row, the dots are separated from each other by a preferably constant distance or pitch and preferably all the rows of dots have the same pitch.

Preferably, the dots of a row are arranged in a median position with respect to the dots of the adjacent row or rows, so that a pair of adjacent dots of one row forms, with the dots of the adjacent row, an isosceles or preferably equilateral triangular motif.

It is preferable that the arrangement adopted tends to maximize the coverage of the bandage surface, approaching the side edges as close as possible. The proximal free edge of the bandage preferably does not exceed 5 mm. By way of example, the density of the dots, understood as the number of dots relative to the surface area of the bandage, may be between 2 and 5 dots/cm².

Moreover, a ratio between free textile surface to surface covered with dots (silicon surface) on the order of 30-50% is preferable and more preferably 35% to 45%.

The dots may be applied to the elastic bandage by means of commercially available siliconing equipment provided via a specific supply chain suitable for obtaining the high density of dots/cm² previously indicated and useful to cover the various heights of commercially available bandages, which may vary within the range of 5 to 12 cm.

According to a further preferred feature of the bandage according to the invention, the fabric of the bandage has a substantially homogeneous weave over its entire extension, i.e., it has no localized regions of weakness.

Preferably, the bandage also has on at least one of its surfaces, and particularly on the surface to which the dots are attached, a plurality of indicator or marker means extending in the longitudinal direction of the bandage, with development parallel to the lateral longitudinal edges and spaced from each other in the transverse direction. In the example shown, the aforementioned indicator means are constituted by a plurality of guidelines, preferably colored, indicated at 10, which may be continuous lines or dashes. The indicator means, for example, may be made of warp threads having a color different from the color of the weave, that is, different from the other warp and weft threads. Such marks or lines may also be made by printing with printing inks on one or both surfaces of the bandage. In particular, such indicator or marker means may also be made as continuous or discontinuous rectilineal motifs, i.e., not just as rectilineal lines but also as, e.g., meander or zigzag lines or with symbols or designs arranged in a rectilineal direction and spaced apart from each other.

The function of said indicator means is to allow a bandaging by wrapping the bandage around the limb with overlapping coils and a constant and reproducible wrap spacing.

In a specific example of embodiment, the bandage has the following features:
- double-weave fabric with the following composition:
   a. 80% cotton weft (27/2 Nm)
   b. 20% polyamide warp (78/2 dtex)
   c. specific weight: approximately 140 g/m² (taut measurement)
   d. heights of the bandages: 5-8-10-12 cm
   e. elongation: approximately 35%
- a silicon covering with dots having a diameter of approximately 0.4 cm with a surface density of about 3 dots/cm² is applied to the fabric.

The free textile surface/siliconized surface ratio is approximately 40%.

The bandaging is carried out by winding the bandage around the affected body part arranging the surface of the bandage whereon the dots are in contact with the patient's skin. As indicated, preferably an elastic compression- or elastic containment-type bandaging is performed.

Due to the features of the bandage object of the invention, the following advantages in particular are realized:
- increased tightness due to the greater surface covered by the elastomeric material (silicone);
- less bulk compared to conventional multilayered short-stretch bandages; due to the presence of the dots, with the same tightness as a corresponding conventional multilayered bandage, fewer coils or a shorter length of bandage is required resulting in less bulk;
- rigidity values that are maintained for a longer time compared to those for a conventional short-stretch bandage: in practice, the bandage does not slip over time and maintains a good compression for a longer period of time;
- greater freedom of movement for the patient compared to that with a conventional multilayered short-stretch bandage;
- the presence of dots allows for a pressure discontinuity (high/low pressure) that stimulates swollen or hardened subcutaneous tissue.

For such reasons, the dotted bandage according to the invention is an active treatment of lymphoedema precisely due to the discontinuous dots on the surfaces of the bandage which exert a differentiated pressure in the edematose zone. In this way, the mobilization of the subcutaneous edematose or fibrous tissue is facilitated and encouraged, in synergy with changes in muscle volume.

Naturally, without altering the principle of the invention, the embodiments and the details of implementation may vary widely with respect to those described and illustrated purely by way of non-limiting example.

## Claims

1. Elastic bandage for realizing an elastic compression or elastic containment bandaging formed of a strip of fabric with longitudinal extension (2), **characterized in that** it comprises on at least one of its surfaces a plurality of punctiform elements (8) of elastomeric material separated and spaced from each other.

2. Elastic bandage according to claim 1, **characterized in that** said punctiform elements (8) are of medical-grade silicone material.

3. Elastic bandage according to claims 1 or 2, **characterized in that** said punctiform elements have a maximum dimension or diameter of 1 mm to 6 mm, preferably from 2 to 5 mm, and an area density of 2 to 5 punctiform elements per cm².

4. Elastic bandage according to any one of claims 1 through 3, **characterized in that** it has a relationship between the free textile surface and the surface covered by said punctiform elements of 30 to 50%, and preferably 35 to 45%.

5. Elastic bandage according to any one of claims 1 through 4, **characterized in that** said punctiform elements are arranged in a row parallel to the longitudinal direction of the bandage and spaced in the transverse direction at a constant pitch.

6. Elastic bandage according to claim 5, **characterized in that** the punctiform elements of a row are arranged in a median position with respect to the punctiform elements of the adjacent row or rows, so that a pair of adjacent punctiform elements of a row forms with a punctiform element of an adjacent row an isosceles or preferably equilateral triangle.

7. Elastic bandage according to any one of the preceding claims, **characterized in that** it is a short-stretch bandage with an elastic elongation of 30% to 40%.

8. Elastic bandage according to any one of the preceding claims, consisting of a double-weave fabric with polyamide warp threads and cotton weft threads.

9. Elastic bandage according to any one of the preceding claims, **characterized in that** it further has on at least one of its surfaces indicator means (10) parallel to the longitudinal edges of the bandage and preferably comprising rectilineal warp threads of a different color than the warp threads of the weave.

10. Elastic bandage according to any one of the preceding claims, for the active treatment of lymphoedema or phlebolymphoedema.
